# EUROPÄISCHE PATENTSCHRIFT

(11) **EP 0 638 297 B1**
(45) Veröffentlichungstag und Bekanntmachung des Hinweises auf die Patenterteilung: **13.01.1999**
(21) Anmeldenummer: 94106517.9
(22) Anmeldetag: 26.04.1994
(51) Int. Cl.: A61C 19/00, A61B 19/00, A61L 2/24, A61L 2/26

(54) **Verfahren und Vorrichtung zur hygienischen Aufbereitung von medizinischen, insbesondere zahnmedizinischen Instrumenten**
Method and device for the hygienic treatment of medical, especially dental instruments
Procédé et dispositif pour le traitement hygiénique d'instruments médicaux, notamment dentaires

(30) Priorität: 15.07.1993 DE 4323815
(43) Veröffentlichungstag der Anmeldung: 15.02.1995
(73) Patentinhaber: Sirona Dental Systems GmbH & Co.KG, 64625 Bensheim (DE)
(72) Erfinder: Hruza, David, Dipl.-Ing., D-88339 Bad Waldsee (DE); Stetter-Alle, Raimund, Dipl.-Ing., D-89073 Ulm (DE); Trackl, Karl, D-89129 Langenau (DE); Sutter, Ralf, Dipl.-Ing., D-69469 Weinheim (DE); Beerstecher, Lutz, Dipl.-Ing., D-64625 Bensheim (DE)
(74) Vertreter: Isenbruck, Günter, Dr.

(56) Entgegenhaltungen:
- EP-A- 0 300 945
- CH-A- 471 583
- DE-A- 3 018 872
- DE-A- 4 024 171
- DE-C- 1 185 348
- US-A- 4 752 444
- US-A- 4 990 087

## Beschreibung

Die Erfindung bezieht sich auf ein Verfahren und eine Vorrichtung zur hygienischen Aufbereitung von medizinischen, insbesondere zahnmedizinischen Instrumenten mit oder ohne Medienkanälen sowie mit oder ohne bewegten Innenteilen.

Zur hygienischen Aufbereitung, insbesondere zahnmedizinischer Instrumente, sind verschiedene Einrichtungen und Verfahren bekannt.

Aus DE-41 30 233, DE-42 11 744 und EP-04 29 960 sind Einrichtungen bekannt, mit denen Instrumente ausschließlich sterilisiert werden können. Eine Reinigung und Pflege (mit Schmiermittel) ist damit nicht möglich.

Aus DE-40 24 171 ist ein Verfahren zur Pflege von insbesondere ärztlichen und zahnärztlichen Instrumenten bekannt. Bei diesem Pflegeverfahren werden die Instrumente in einem ununterbrochenen Verfahrensablauf und an einem einzigen, alle Verfahrensschritte erlaubenden Pflegeplatz folgenden Verfahrensschritten unterzogen:

Außenreinigung, Desinfektion, Innentrocknung und Pflege, Nachreinigung sowie Außentrocknung und Abkühlung.

Die zu pflegenden Instrumente werden hierzu in eine Spülkammer getaucht, wo sie den vorgenannten Verfahrensschritten unterzogen werden. Die Außenreinigung erfolgt dabei zunächst durch Ausblasen der Instrumente mittels Kaltluft, wobei äußerlich eine Spülung durch das Wasser erfolgt, welches durch eingeblasene Luft in Bewegung kommt. Danach wird Heißwasser in die Spülkammer eingelassen; die Instrumente werden sodann im Wasserbad ausgekocht. Auf diesen Verfahrensschritt folgt eine Innentrocknung und Pflege der Instrumente. Dazu werden sie mit Heißluft ausgeblasen, wobei durch die Heißluft Öl in die Instrumente eingeblasen wird. Die darauffolgende Nachreinigung besteht in der Entfernung überschüssigen Öls im Instrumenteninneren durch Einblasen von Heißluft mit erhöhtem Druck. Außerdem werden die Instrumente nochmals mit Heißwasser behandelt, welches in die Spülkammer eingeleitet wird. Die Trocknung erfolgt durch Eigenwärme der Instrumente. Zum Abkühlen wird Kaltluft in den Spülraum geblasen. Die gesamte Verfahrensdauer beträgt ca. 20 Minuten.

Abgesehen davon, daß, um den Spülkammerinhalt zu erhitzen, eine relativ lange Aufheizzeit erforderlich ist, benötigt man bei diesem bekannten Verfahren vergleichsweise viel Wasser und damit viel Energie. Des weiteren ist, um einen akzeptablen Reinigungseffekt zu erzielen, die Beigabe von Tensiden (Reinigungsmittel) erforderlich. Ein weiterer Nachteil ist darin zu sehen, daß mit diesem Verfahren behandelte Instrumente, sollen sie steril sein, anschließend noch sterilisiert werden müssen, beispielsweise in einem Autoklaven der eingangs genannten Gattung.

Aus der CH-A-471 583 ist weiterhin eine Einrichtung zur Reinigung und Sterilisation medizinischer Instrumente bekannt, bei der die Instrumente zunächst in einen perforierten Behälter gelegt werden, der dann in eine dicht abschließbare Kammer gegeben wird. In der Kammer befinden sich, ähnlich einer Haushaltsspülmaschine, mehrere Spülarme, die durch einen Hydroantrieb alternierend hin- und herbewegt werden. Die Spülarme sind mit einer Vielzahl von Düsen belegt, über die Reinigungswasser und/oder Dampf in die Kammer geleitet werden kann. Die Instrumente können so gereinigt und/oder sterilsiert werden. Der Ablauf des Reinigungs- bzw. Sterilisationsprozesses kann mit Hilfe einer auswechselbaren Programmkarte individuell gesteuert werden. Die Zufuhr von Pflegemitteln ist mit dieser Einrichtung nicht möglich.

In der DE-A1-30 18 872 ist eine Vorrichtung zum Reinigen von Gegenständen, insbesondere eine Spülmaschine für medizinische Geräte beschrieben, bei der Reinigungsflüssigkeit intermitierend auf die Gegenstände gespritzt wird, wobei die Pausen zwischen den Spritzvorgängen so lang sind, daß ein auf den Gegenständen gebildeter Film der Reinigungsflüssigkeit abgerissen ist, bevor ein weiterer Spritzvorgang beginnt. Die zu reinigenden Gegenstände werden einerseits von oben über einen mit Spritzdüsen besetzten Dreharm und andererseits von unten über sogenannte Langdüsen, die sich in das Innere der zu reinigenden Gegenstände erstrecken, besprüht. Das Einspritzen der Reinigungsflüssigkeit erfolgt mittels Druckluft.

In der DE-A1-11 85 348 wird schließlich ein Wasserspritzverfahren zum Spülen von Geschirr beschrieben, bei dem das Wasser unter Ausnutzung von Druckluft periodisch aus mehreren Rohren gepreßt wird. Die Druckluft dient dazu, die über die Wasserleitung zugeführten, in den Rohren befindlichen Wasserstangen auszustoßen. Das hier angewandte Verfahren besteht also darin, abwechselnd die Rohre mit einer bestimmten Wassermenge zu beladen und dieses Wasservolumen dann mit Hilfe von Druckluft auszustoßen.

Aufgabe der vorliegenden Erfindung ist es, ein gegenüber dem Stand der Technik verbessertes Verfahren und eine danach arbeitende Vorrichtung anzugeben, mit der Zielsetzung, die hygienische Aufbereitung medizinischer, insbesondere zahnmedizinischer Instrumente effizienter und wirkungsvoller durchführen zu können.

Ein wesentlicher Effekt liegt in der Reinigung der Außenflächen der Instrumente.

Wie Versuche gezeigt haben, wird mit den so alternierend dem Wasser zugeführten Gasimpulsen ein sehr wirksamer Reinigungseffekt erzielt. Ein weiterer Vorteil besteht darin, daß, wenn der zur Verfügung stehende Wasserdruck schwankt bzw. niedriger ist, dann durch das stoßweise Zuführen des Gases der Energiegehalt des Reinigungsmediums (Wasser/Luft-Gemisch) erhalten bleibt. Der Reinigungseffekt ist somit weitgehend unabhängig vom zur Verfügung stehenden Wasserdruck.

Die Impulsfrequenz für das stoßweise Zuführen des Gases liegt vorteilhafterweise bei etwa 3 Hz.

Gemäß einer vorteilhaften Ausgestaltung des erfindungsgemäßen Verfahrens erfolgt im Anschluß an den Verfahrensschritt 2 eine Zwischentrocknung mittels eines Gases, vorzugsweise mittels Druckluft.

Das Pflegen erfolgt vorteilhafterweise mittels eines Öl-Luft-Gemisches.

Um insbesondere aus Hohlräumen der Instrumente die dort vorhandene Trockenluft zu entfernen, ist es vorteilhaft, die Sterilisationskammer vor der Sterilisation zu evakuieren. Des weiteren ist es vorteilhaft, den Vorgang des Trocknens durch Vakuum zu unterstützen.

Um dem Anwender die Möglichkeit zu bieten, Instrumente, die nicht unbedingt sämtlichen Verfahrensschritten unterworfen werden müssen, bei Bedarf aus dem Gerät entnehmen zu können, sind Mittel vorgesehen, mit denen das Verfahren zumindest nach dem Pflegeschritt 3 unterbrochen werden kann. Desgleichen kann es vorteilhaft sein, einen der Verfahrensschritte, z.B. den Verfahrensschritt der Sterilisation, gegebenenfalls zu überspringen bzw. wegzulassen.

Das erfindungsgemäße Verfahren sowie ein Ausführungsbeispiel eines Gerätes zur Durchführung des Verfahrens werden nachfolgend anhand der Zeichnung näher erläutert.

Es zeigen:
Figur 1 eine Ausführungsform der erfindungsgemäßen Vorrichtung in einer schaubildlichen Darstellung,
Figur 2 eine Ausführungsform einer in die Vorrichtung nach Figur 1 einsetzbaren Instrumentenkassette in schaubildlicher Explosionsdarstellung,
Figuren 3 und 4 eine Ausführungsform einer Abdeckung an der Rückseite der in Figur 2 gezeigten Kassette,
Figur 5 einen der in Figur 2 gezeigten Adapter und den rückwärtigen Teil eines dazu passenden Instruments im Längsschnitt,
Figur 6 eine vereinfachte Darstellung der Anordnung einer mit Instrument bestückten Kassette im Gerät.
Figur 7 ein hydraulisch/pneumatisches Schaltbild.
Figur 8 ein Impuls-Zeitdiagramm,
Figur 9 eine Variante zu der in Figur 6 gezeigten Ausführungsform.

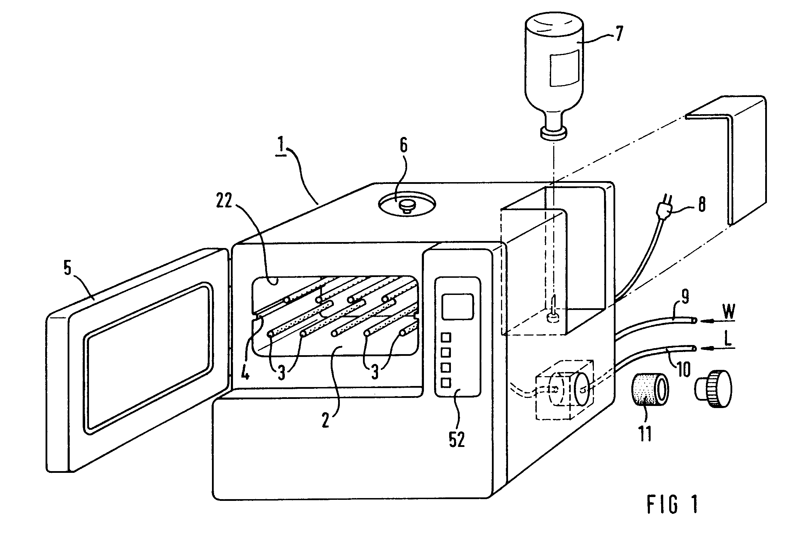

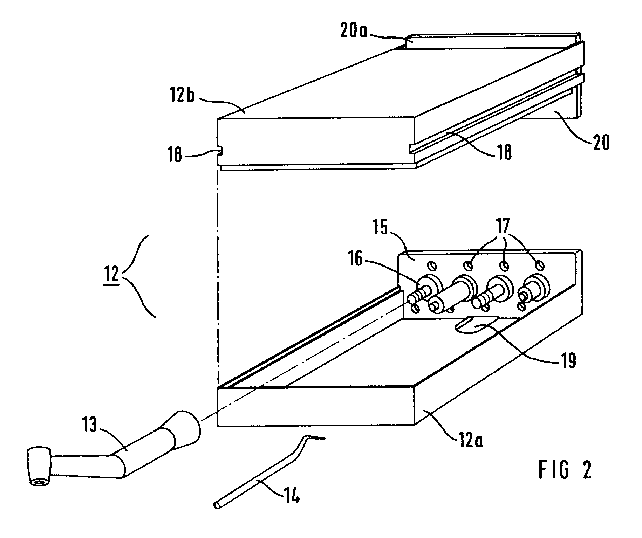

Die Figur 1 zeigt in einer schaubildlichen Darstellung eine Ausführungsform des erfindungsgemäßen Gerätes, welches hier zur hygienischen Aufbereitung diverser zahnmedizinischer Instrumente dient. Das Gerät enthält ein allgemein mit 1 bezeichnetes Gehäuse, welches frontseitig eine Kammer 2 zur Aufnahme einer später noch näher erläuterten Kassette bildet. In die Kammer 2 ragen eine Vielzahl (hier neun) von Düsenarmen 3, mit deren Hilfe, wie ebenfalls später noch näher erläutert, die Instrumente außen besprüht werden können. Beidseitig der Kammer 2 sind Führungsleisten 4 zur Führung der in Figur 2 näher dargestellten Kassette angeordnet. Die Kammer 2 ist in bekannter Weise frontseitig mittels eines Deckels 5 druckdicht verschließbar.

Im rückwärtigen Teil des Gehäuses 1 befindet sich an der Oberseite die Einfüllöffnung 6 eines nicht dargestellten Behälters für demineralisiertes bzw. destilliertes Wasser. Seitlich daneben ist eine abdeckbare Nische zur Aufnahme eines Schmierölvorratsbehälters 7 vogesehen. Dieser Behälter kann in bekannter Weise einen Membranverschluß aufweisen, der beim Aufsetzen auf einen entsprechend ausgebildeten Anschlußzapfen von diesem durchstochen wird, wodurch eine Entnahme des Schmieröls ermöglicht ist. Mit 8, 9 und 10 sind Zuleitungen für elektrische Energie, Druckwasser sowie Druckluft bezeichnet. Mit 11 ist ein leicht entnehmbares und damit austauschbares Sterilfilter bezeichnet, welches im Leitungskanal der Druckluftzuleitung einsetzbar ist. Damit kann sichergestellt werden, daß weitgehend keimfreie Luft für die nachfolgend näher erläuterten Verfahrensschritte zur Verfügung steht.

Die Figur 2 zeigt in einer schaubildlichen Explosionsdarstellung eine in die Kammer 2 einsetzbare Kassette 12 zur Aufnahme mehrerer Instrumente 13 und 14, die hygienisch aufbereitet werden sollen. Das in der Figur mit 13 bezeichnete Instrument ist ein Hand- und Winkelstück, welches im Innern diverse Antriebsteile und Lager sowie Medienkanäle aufweist. Das Instrument 14 dagegen ist ein Handinstrument, welches keine bewegten Teile und auch keine Medienkanäle aufweist.

Die Kassette 12 besteht aus einem Basis- oder Unterteil 12a und einem abnehmbar daran gehalterten Kassettenoberteil 12b. Das Kassettenunterteil 12a ist wannenförmig ausgebildet und enthält an einer erhöhten Rückwand 15 vier verschiedene Adapter zur Aufnahme unterschiedlich gestalteter Instrumente. Im dargestellten Ausführungsbeispiel ist der Adapter 16 auf das aufzunehmende Instrument 13 abgestimmt. Die vier Adapter sind für Instrumente vorgesehen, die bewegte Innenteile und Medienkanäle aufweisen, wie z.B. Hand- und Winkelstücke, Turbinen-, Zahnsteinentfernungs- oder Spritzhandstücke. Instrumente, die keine bewegten Innenteile haben und auch keine Medienkanäle beinhalten, wie beispielsweise das in der Figur mit 14 bezeichnete Handinstrument, werden zur Aufbereitung mittels geeigneter Halterungen am Boden des Kassettenunterteils plaziert.

Die Rückwand 15 des Kassettenunterteils 12a enthält (neun) Bohrungen 17, durch die beim Einsetzen der Kassette 12 in die Kammer 2 die dort vorhandenen (neun) Düsenarme 3 hindurchgreifen können. Die Anordnung der Düsenarme 3 und der mit ihnen korrespondierenden Bohrungen 17 sind, wie aus der Abbildung hervorgeht, jeweils dreieckförmig um die mittig gelegenen Adapter angeordnet.

Das auf das Kassettenunterteil 12a aufsetzbare und mit diesem rastend verbindbare Oberteil 12b enthält seitliche Führungsnuten 18, die mit den bereits erwähnten Führungsleisten (Pos. 4 in Fig. 1) zusammenwirken und eine exakte Zentrierung und Führung der Kassette in der Kammer 2 gewährleisten.

Die Kassette ist an geeigneter Stelle, vorzugsweise bodenseitig und dicht benachbart der Rückwand 15 mit einer oder mehreren Ablauföffnungen 19 für den Ablauf des zugeführten Reinigungs- sowie Kondenswasser versehen.

Vorteilhaftweise ist (sind) diese Ablauföffnung(en) mit einem beim Entnehmen der Kassette selbsttätig verschließenden Teil, z.B. mit einem federbelasteten Schieber oder Deckel, versehen.

Das Kassettenoberteil 12b ist vorteilhafterweise an der Rückseite mit einer Abdeckung versehen sein, welche die Öffnungen 17 und die zu den Adaptern 16 führenden Anschlüsse sowie gegebenenfalls die Ablauföffnung 19 abdeckt bzw. verschließt, wenn die Kassette aus dem Gerät herausgenommen wird.

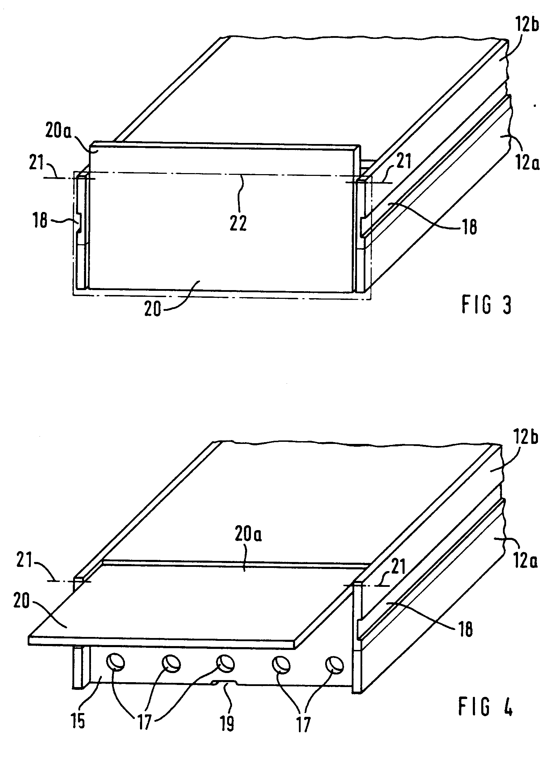

Die Figuren 3 und 4 zeigen eine Ausführungsform einer solchen Abdeckung, wobei die Figuren die Kassette von der Rückseite zeigen. Eine Abdeckplatte 20 ist um ein Lager 21 nach oben aufklappbar. Das Aufklappen geschieht automatisch beim Einschieben der Kassette 12 in die Kammer 2 des Gerätes 1. Hierzu weist die Abdeckplatte 20 einen Überstand 20a auf, der beim Einschieben der Kassette in die Kammer 2 an der mit 22 angedeuteten Oberkante der Kammer 2 anschlägt, wodurch die Klappe nach oben geöffnet wird (Fig. 4). Die sich dahinter befindlichen Öffnungen 17 sowie die aus der Abbildung nicht ersichtlichen Anschlüsse zu den Adaptern 16 werden sodann freigegeben. Beim Herausnehmen der Kassette klappt die Abdeckplatte 20 automatisch wieder nach unten und verschließt so die Öffnungen, so daß die in der Kassette befindlichen, hygienisch aufbereiteten Instrumente gegen Eindringen von Keimen zumindest für einen bestimmten Zeitraum geschützt sind.

Alternativ zu der aufgezeigten, selbsttätig öffnenden und schließenden Klappe kann auch ein Rolladenelement vorgesehen werden, welches mit Hilfe entsprechend vorgesehener Stellmittel eine selbsttätige Abdeckung bzw. Freihaltung der an der Rückwand 15 vorgesehenen Öffnungen bzw. Anschlüsse bewirkt.

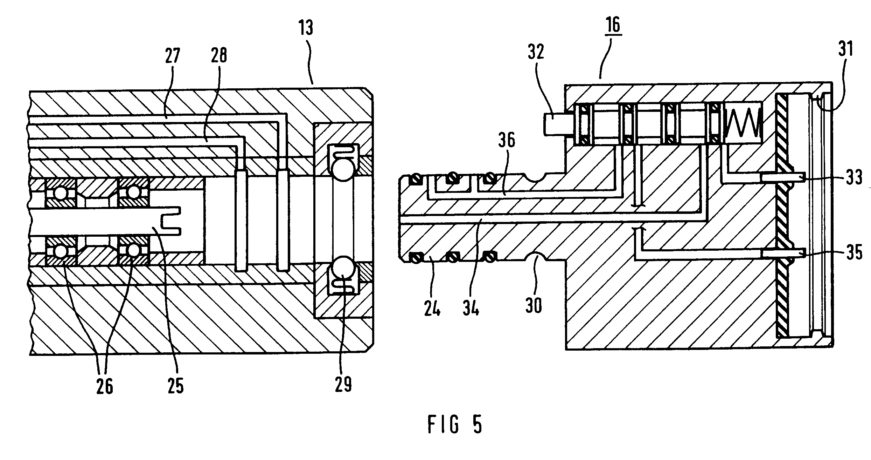

Anhand der Figur 5 wird am Beispiel des Adapters 16 der Aufbau und die Funktion der Adapter sowie das Zusammenwirken mit den daran aufsteckbaren Instrumenten erläutert.

Der Adapter 16 ist hier zum Anschließen eines in Figur 2 mit 13 bezeichneten Hand- und Winkelstückes vorgesehen, welches in seinem Inneren in Rotation versetzbare Triebwellen 25 sowie diesen zugeordnete Lager 26 enthält. Des weiteren verlaufen im Inneren eines solchen Handstückes Medienkanäle 27, 28 zur Zuführung von Kühlluft und Kühlwasser an die Präparationsstelle. Mit 29 ist eine Kugelrasteinrichtung bezeichnet, die beim Aufsetzen des Instruments 13 an einen diesem zugeordneten Antrieb mit einer entsprechenden Ringnut korrespondiert. Eine solche Ringnut ist beim Adapter 16 vorgesehen und dort mit 30 bezeichnet.

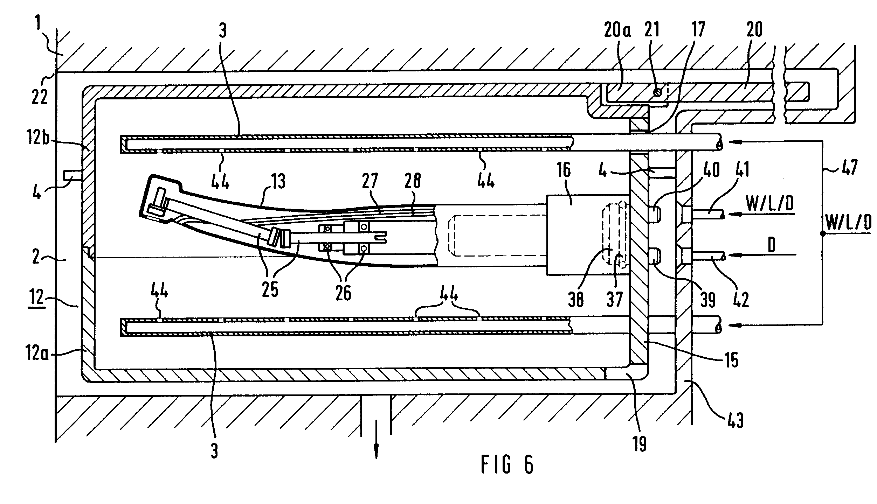

Der Adapter 16 enthält einen Anschlußzapfen 24, der baulich auf die Konstruktion des Instruments abgestimmt ist. An dem dem Instrument ab gewandten Ende ist der Adapter 16 so ausgebildet, daß er an der Rückwand 15 des Kassettenunterteils 12a leicht lösbar befestigt werden kann. Hierzu enthält er eine Rastnase 31, die mit einer Ringnut 37 eines Flansches 38 (Fig. 6) an der Rückwand 15 des Kassettenunterteils 12a zusammenwirkt.

Im Adapter 16 befindet sich ein achsparallel verschiebbarer Schaltplunger 32, welcher beim Aufschieben des Instruments 13 entgegen der Kraft einer nicht näher bezeichneten Feder betätigt wird. Bei Betätigung wird der Eingangskanal 33 mit einem zentrisch gelegenen Kanal 34 verbunden. Außerdem wird die Verbindung eines weiteren Eingangskanales 35 mit einem Leitungskanal 36 hergestellt, der im aufgesetzten Zustand des Instruments über periphere Öffnungen mit den Medienleitungen 27, 28 des Instruments verbunden ist. Über den Eingangskanal 33 kann Treibluft oder Schmieröl zu den Triebwellen 25 und deren Lager 26 geleitet werden; über den Leitungskanal 35 kann Luft und Wasser gemeinsam zu den Leitungen 27 und 28 geführt werden.

Mit dem Plungerventil 32 ist sichergestellt, daß bei nicht mit einem Instrument belegtem Adapter die zugeführten Medien am Adapter nicht austreten können.

Wie bereits erwähnt, sind die Anschlußmaße bezüglich der Instrumente für alle vier, in der Kassette gehalterten Adapter unterschiedlich, um so unterschiedliche Instrumente haltern zu können. Die Anschlußmaße bezüglich des Anschlusses an der Rückwand 15 der Kassette sind jedoch für alle Adapter gleich, so daß sie sehr leicht untereinander ausgetauscht bzw. gegen solche mit anderen Anschlußkonturen für andere Instrumente gewechselt werden können.

Anhand der Figur 6 wird am Beispiel des Instruments 13 und des Adapters 16 die Zuordnung der einzelnen Wasch- und Reinigungsdüsen bzw. -kanäle erläutert.

Die Figur 6 zeigt in einer stark vereinfachten Darstellung die Anordnung der Kassette 12 in der Kammer 2 in nahezu vollständig eingeschobenem Zustand. An der Rückwand 15 der Kassette 12 befinden sich, entsprechend der Anzahl der vorhandenen Adapter, Anschlußflansche 38, auf die, wie bereits erwähnt, die Adapter 16 leicht lösbar aufgesetzt werden können. Die Anschlußflansche 38 weisen Verbindungsmuffen 39, 40 auf, welche bei völlig eingeschobener Kassette eine Verbindung herstellen, einerseits zwischen Zuleitungen 41, 42 und den bereits erwähnten Eingangskanälen 33 und 35 (Fig. 5). Die um das Instrument 13 stern- bzw. dreieckförmig herum angeordneten Düsenarme 3 sind an der Gehäusewandung 43, welche die Kammer 2 rückseitig begrenzt, fest angeordnet. Sie sind als Hohlleitungen ausgebildet und weisen eine Vielzahl von Düsenaustrittsöffnungen 44 auf, die unter einem bestimmten Winkel auf die Oberfläche des Instruments 13 ausgerichtet angeordnet sind. Die Düsenbohrungen sind vorteilhafterweise längs der Düsenarme nicht nur in einer Ebene, sondern in mehreren um einen bestimmten Winkel gegeneinander versetzten Ebenen angeordnet. Der Versatz kann vorteilhafterweise zick-zack- oder wellenförmig in Längsrichtung entlang einer Bezugsebene angeordnet sein.

Wie aus Figur 1 und auch aus dem nachfolgend noch näher erläuterten hydraulisch-pneumatischen Schaltplan hervorgeht, sind zur Aufbereitung von maximal vier Instrumenten vorteilhafterweise neun Düsenarme vorgesehen. Die Anordnung ist dabei so getroffen, daß die Düsenarme jeweils in einem Winkel von 120° zueinander um ein Instrument herum angeordnet sind.

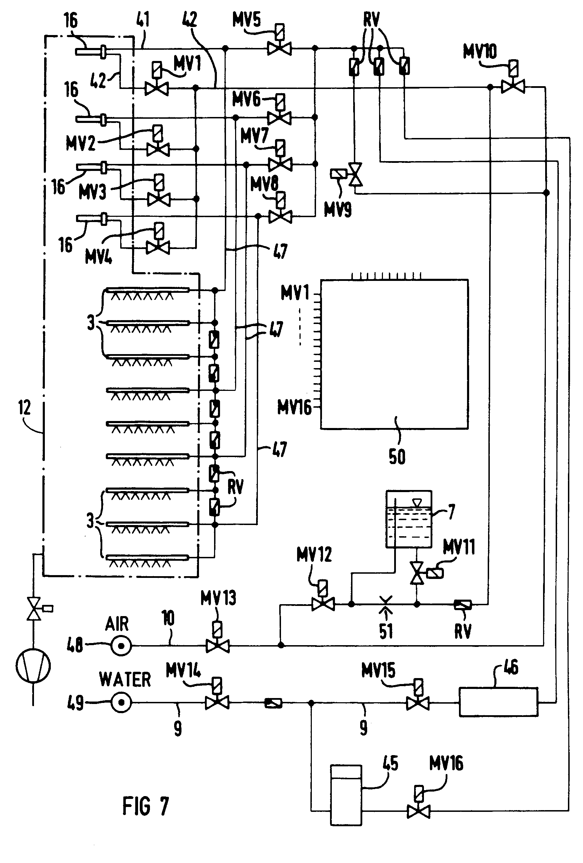

Bevor das erfindungsgemäße Verfahren anhand der bereits erläuterten Zeichnung und des hydraulisch-pneumatischen Blockschaltbildes gemäß Figur 7 näher erläutert wird, sei noch erwähnt, daß im Gerätegehäuse außer dem bereits erwähnten Ölbehälter 7 auch ein an die Druckwasserleitung 9 angeschlossener Dampferzeuger 45 sowie eine Vorwärmeinrichtung 46 für Wasser untergebracht sind.

Die Figur 7 zeigt ein hydraulisch-pneumatisches Schaltbild von der erfindungsgemäßen Einrichtung.

Mit 48 und 49 sind die Versorgungsquellen bezeichnet, mit denen einerseits Druckluft und andererseits Druckwasser in das Gerät eingespeist werden. Die eingangsseitig üblichen Filter, Ölabscheider (bei Druckluft) sowie Überdruckventile sind der Einfachheit halber nicht eingetragen.

Sämtliche Magnetventile (MV1 bis MV16) sowie die Wasserheizung 46 und der Dampferzeuger 45 werden von einem Mikroprozessor 50 aus gesteuert. Mit RV sind in die Leitungen geschaltete Rückschlagventile bezeichnet.

Die Injektion des Schmiermittels (mit Luft vermischbares Pflegeöl) aus dem Behälter 7 erfolgt hier pneumatisch über die beiden Magnetventile MV1 und MV 12 sowie eine Droselstelle 51. Alternativ kann das Schmieröl auch mittels einer geeigneten Ölpumpe zugeführt werden. Der MP 50 steuert die Magnetventile MV1 bis MV9 und MV11 bis MV16 so, daß den Adaptern 16 und den Düsenarmen 3 die Medien Luft, Wasser und gegebenenfalls das Schmieröl bei den Verfahrensschritten der Vor- und der Nachreinigung - und gegebenenfalls beim Pflegevorgang - für jedes Instrument getrennt zugeführt werden, so daß die Instrumente zeitlich nacheinander gereinigt - und gegebenenfalls gepflegt - werden.

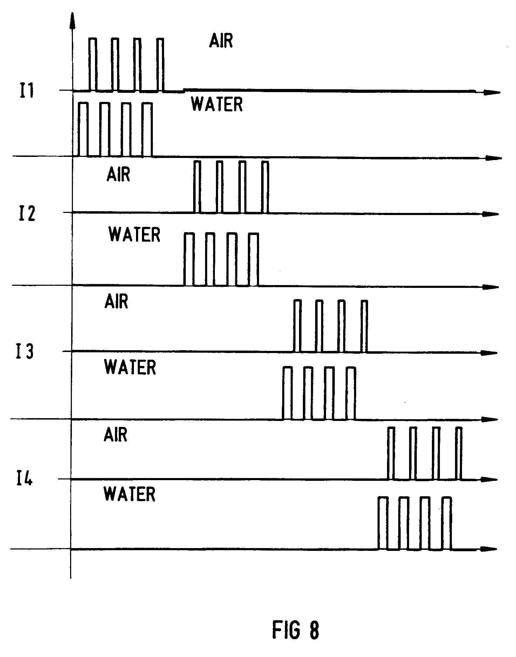

Aus Figur 8, die den zeitlichen Verlauf der Ventilanschaltung für die Medien Luft und Wasser für vier Instrumente (im Diagramm mit I1 bis I4 bezeichnet) aufzeigt, ist dieser zeitliche Versatz erkennbar. Mit dieser zeitlich aufeinanderfolgenden Zuschaltung der Medien wird bei niedrigem Wasserverbrauch eine hohe Reinigungseffizienz erzielt.

Das erfindungsgemäße Verfahren läuft wie folgt ab:

Zunächst wird die Kassette 12 mit Instrumenten beschickt. Instrumente, die bewegbare Innenteile haben, wie Winkelstücke, Turbinen, Spritzen, od.dgl., werden auf die entsprechenden Adapter 16 (max. vier) der Kassette aufgesteckt. Die übrigen Instrumente werden an Haltevorrichtungen über dem Behälterboden plaziert. Die Kassette wird sodann durch Aufsetzen des Kassettenoberteils verschlossen und in die Kammer 2 des Gerätes eingeführt. Mit dem Einführen wird die rückwärtige Abdeckung 20 angehoben, wodurch die neun Düsenarme 3 durch die Bohrungen 17 der Kassettenrückwand hindurchgreifen können. Nachdem die Kassette in der Kammer 2 so weit eingeschoben ist, daß die Verbindung der Leitungen 41, 42 mit den Anschlüssen 39, 40 (Fig. 6) hergestellt ist, wird der Deckel 5 das Gerät geschlossen und das Gerät eingeschaltet. Nach einem Selbsttest, der etwa eine halbe Minute dauert, erfolgt zunächst eine Kaltreinigung der Instrumente, indem über die Zuleitung 9 und die Leitungen 41 und 47 den Adaptern 16 und den Düsenarmen 3 kaltes Wasser zugeführt wird. Das kalte Wasser wird pulsierend zugeführt, wobei dem Wasser in den Impulspausen Druckluft mit höherem Druck (ebenfalls pulsierend) beigemischt wird (Fig. 8). Dadurch wird ein hochenergetischer Masserstrahl erzeugt, der überraschenderweise eine sehr gründliche Reinigung der Oberfläche und auch der Innenkanäle bewirkt. Gleichzeitig wird über die Leitung 42 der Getriebekanal mit Luft beaufschlagt (das Ölzuführungsventil MV11 bleibt geschlossen), wodurch das Eindringen von Schmutz und (verschmutztem) Reinigungswasser verhindert wird. Die Impulsfrequenz, mit der dem Wasser Druckluft zugeführt wird, beträgt etwa 3 Hz.

Die Kaltreinigung ist nach etwa 2 Minuten beendet. Danach erfolgt eine Warmreinigung der Außenflächen der Instrumente und auch der Innenkanäle, ebenfalls mit einem pulsierenden Wasserstrahl, unter Beifügung von gepulster Druckluft. Dieser Vorgang läuft wie vorerwähnt ab. Nach etwa 2 Minuten Warmreinigung erfolgt eine intensive Nachreinigung und Dampfdesinfektion durch Ab-und Ausblasen der Außenflächen der Instrumente und der Medien-und Getriebekanäle im Innern der Instrumente mit Heißwasser von 60° bis 100°C. Das Wasser wird hierzu von dem als Durchlauferhitzer wirkenden Erhitzer 46 erwärmt. Dieser Vorgang dauert etwa 30 bis 45 Sekunden.

Nach dieser intensiven Nachreinigung erfolgt eine Pflege der bewegten Innenteile durch Injektion einer dosierten Menge Schmieröl aus dem Schmierölvorratsbehälter 7. Die Zudosierung kann in Abhängigkeit vom adaptierten Instrument erfolgen und beträgt im Mittel 1 Gramm pro Instrument und Injektionszyklus.

Dieser Pflegeschritt dauert etwa eine halbe Minute. Anschließend erfolgt eine Sterilisation der Instrumente, und zwar sowohl von außen als auch von innen, mittels gesättigtem Wasserdampf von vorzugsweise 134°C bei einem Druck von etwa 2 bis 2,5 bar. Der Sterilisationsvorgang beträgt zwischen 5 und 6 Minuten. Danach folgt ein Trocknen und Abkühlen der Instrumente mittels kalter Luft. Der Abkühlvorgang dauert etwa 3 bis 5 Minuten. Bei einer Gesamtzeit von etwa 15 bis 20 Minuten zur Aufbereitung der Instrumente kann danach die Kassette mit den aufbereiteten Instrumenten entnommen und im geschlossenen Zustand zur Benutzung bereitgestellt werden.

Der Verfahrensablauf, der an sich selbsttätig in der geschilderten Weise abläuft, kann durch Eingriff, z.B. durch entsprechende Programmvorwahl, die an einem Bedien- und Displayfeld 52 an der Frontseite des Gerätes abgerufen werden kann, variiert werden, z.B. indem im Anschluß an die intensive Nachreinigung und Desinfektion der Außenfläche eine Zwischentrocknung, z.B. durch Luft, durchgeführt wird. Ebenso kann nach dem Pflegen eine Unterbrechung stattfinden, um so nicht sterilisierbare Gegenstände aus der Kassette entnehmen zu können.

Zur Entfernung vorhandener Trockenluft, insbesondere aus Hohlräumen, und im besonderen aus Hohlräumen der Instrumente, kann gemäß einer vorteilhaften Variante die Kammer vor dem Sterilisieren der Instrumente evakuiert werden. Um eine optimale Entfernung der Trockenluft zu erzielen, sollte das Vakuum im Bereich zwischen 40 und 500 mbar absolut liegen. Das Vakuum kann nach dem an sich bekannten fraktionierten (mehrstufigen) Verfahren oder nach dem Vorvakuumverfahren erzeugt werden.

Mit dem geschilderten Verfahren ergeben sich extrem kurze Zeiten für eine optimale hygienische Aufbereitung der Instrumente. Durch den pulsierenden Wasserstrahl, dem stoßweise Druckluft höherer Energie zugeführt wird, ist eine besonders intensive Reinigung der Teile gewährleistet. Dadurch, daß nach der Aufbereitung die Instrumente in der Kassette verbleiben können, ist ein sicherer Transport zum Behandlungsplatz gegeben. Das gleiche gilt für den Weg nach Benutzung der Instrumente zur Aufbereitung. Die nach der Benutzung mit Keimen kontaminierten Instrumente verbleiben bis zur Wiederentnahme in der Kassette, wodurch u.a. eine Verletzungsgefahr für das Bedienpersonal ausgeschlossen ist. Bearbeitung, Lagerung und Transport erfolgen somit in einer Kassette. Nachdem die Kassette weitgehend abgeschlossen ist, ist eine sterile Lagerung der Instrumente zumindest über einen hinreichend langen Zeitraum (Stunden) gewährleistet. Dadurch, daß die Innenteile und die Medienkanäle mit Heißwasser und Dampf durchströmt werden, ergibt sich eine relativ rasche Aufheizzeit der gesamten Instrumente. Durch das pulsierende Ab- und Ausblasen der Teile mit Wasserdampf wird auch ein gewisser kalklösender Effekt in den Leitungen erzielt. Ein wesentlicher Vorteil ist, daß keinerlei Reinigungs- und Treibmittel verwendet zu werden brauchen.

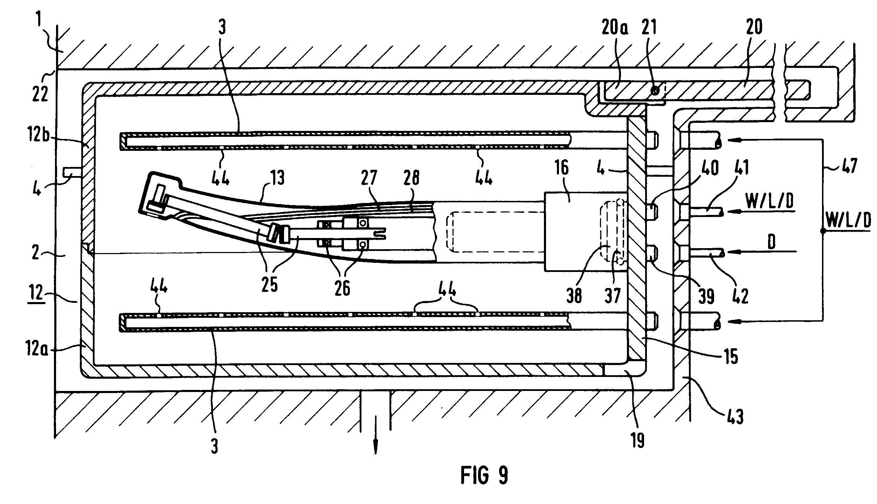

Hinsichtlich der Anordnung und Ausbildung der Düsenarme 3 sind im Rahmen der Erfindung verschiedene Modifikationen denkbar. So können beispielsweise die Düsenarme 3 auch Bestandteil der Kassette sein, wie dies im Ausführungsbeispiel nach Fig. 9 dargestellt ist. Des weiteren könnten, obgleich dies relativ aufwendig wäre, die Düsenarme um ihre Längsachse schwenkbar und gegebenenfalls auch in Achsrichtung bewegbar angeordnet sein. Die in den Fig. 3 bis 5 dargestellte Abdeckung kann alternativ auch Bestandteil des Kassettenunterteils sein oder beiden Kassettenhälften zugeordnet sein.

## Patentansprüche

1. Verfahren zur hygienischen Aufbereitung von medizinischen, insbesondere zahnmedizinischen Instrumenten mit oder ohne Medienkanälen und mit oder ohne bewegbaren Innenteilen, bei dem in einem einzigen Gerät (1) und in einer druckdicht verschließbaren Kammer folgende Verfahrensschritte in einem vorwählbaren Prozeß automatisch ablaufen:
1.1 Reinigen der Außenflächen der Instrumente (13, 14) sowie gegebenenfalls der Medienkanäle (27, 28) mittels eines gezielt auf die Instrumente gerichteten hochenergetischen Wasserstrahles, zunächst mit Kaltwasser, anschließend mit vorgewärmten Wasser, wobei der Wasserstrahl stoßweise zugeführt wird und wobei dem Wasser in den Impulspausen ein Gas unter höherem Druck beigemischt wird,
1.2 intensive Nachreinigung und Desinfektion der Außerflächen und gegebenenfalls der Medienkanäle (27, 28) sowie der bewegbaren Innenteile (25) und deren Lager (26) durch Ab- und Ausblasen mittels Wasserdampf von 60 bis 100°C.
1.3 Pflege der bewegbaren Innenteile (25) und deren Lager durch Injektion einer dosierten Menge eines Schmiermittels,
1.4 Sterilisation der Instrumente (13, 14) außen und innen mittels gesättigtem Wasserdampf.
1.5 Trocknen und Abkühlen der Instrumente (13, 14) mittels eines Kühlmediums.

2. Verfahren nach Anspruch 1, bei dem im Anschluß an den Verfahrensschritt 1.2 eine Zwischentrocknung mittels eines Kühlmediums erfolgt.

3. Verfahren nach Anspruch 1, bei dem das Pflegen mittels eines Öl/Luftgemisches erfolgt.

4. Verfahren nach Anspruch 3, bei dem die Ölmenge in Abhängigkeit von dem adaptierten Instrument (13) automatisch gewählt wird.

5. Verfahren nach Anspruch 3, bei dem die Ölmenge zwischen 0,1 und 2 Gramm pro Instrument und Injektionszyklus liegt.

6. Verfahren nach Anspruch 1, bei dem durch einen Eingriff in den Verfahrensablauf dieser nach dem Verfahrensschritt 1.3 unterbrochen werden kann.

7. Verfahren nach Anspruch 1, bei dem durch einen Eingriff in den Verfahrensablauf der Verfahrensschritt 1.4 übersprungen werden kann.

8. Verfahren nach Anspruch 1, bei dem das Trocknen und Abkühlen mittels Druckluft erfolgt.

9. Verfahren nach Anspruch 1, bei dem vor der Sterilisation der Behandlungsraum evakuiert wird.

10. Verfahren nach Anspruch 1, bei dem das Trocknen der Instrumente durch Vakuum unterstützt wird.

11. Verfahren nach einem der vorangegangenen Ansprüche, bei dem der Gesamtzyklus vorzugsweise im Bereich zwischen 10 und 20 Minuten liegt, wobei die Einzelzyklen für
den Verfahrensschritt 1.1 bei 3 Minuten
den Verfahrensschritt 1.2 bei 2 Minuten
den Verfahrensschritt 1.3 bei 0,5 Minuten
den Verfahrensschritt 1.4 bei 6,5 Minuten
den Verfahrensschritt 1.5 bei 3 Minuten im Mittel liegen.

12. Verfahren nach Anspruch 1, bei dem die Impulsfrequenz für das stoßweise Zuführen des Gases zwischen 1 Hz und 10 Hz liegt, vorzugsweise 3 Hz beträgt.

13. Verfahren nach einem der vorangegangenen Ansprüche, bei dem zumindest die Verfahrensschritte 1.1 und 1.2 und gegebenenfalls der Verfahrensschritt 1.3 für jedes Instrument, vorzugsweise für Instrumente (13) die Medienkanäle (27, 28) und bewegte Innenteile (25, 26) aufweisen, getrennt zeitlich nacheinander ablaufen.

14. Gerät zur Durchführung des Verfahrens nach einem der Ansprüche 1 bis 13, bei dem in der druckdicht verschließbaren Kammer (2), eine ortsfest angeordnete Düsenanordnung mit einer Vielzahl von um die Instrumente (13, 14) herum angeordneten und auf die Außenflächen der Instrumente gerichteten Düsen (44) vorgesehen ist und welches einen Mikroprozessor (50) aufweist, der die Wasser- und Gaszufuhr so steuert, daß die Wasserzufuhr pulsierend erfolgt und daß dem Wasser in den Impulspausen ein Gas unter höherem Druck beigemischt wird.

15. Gerät nach Anspruch 14, enthaltend eine die Instrumente (13, 14) aufnehmende Kassette (12), die in die verschließbare Kammer (2), in der der Verfahrensprozeß abläuft, einlegbar ist.

16. Gerät nach Anspruch 15, bei dem die Kassette (12) zumindest für Instrumente (13), die Medienkanäle (27, 28) und gegebenenfalls bewegbare Innenteile (25) aufweisen, Adapter (16) vorhanden sind, welche Mittel (24) zur Halterung der Instrumente sowie Mittel (33 bis 36) zur Zufuhr von fluiden- und gasförmigen Medien zur Behandlung der bewegbaren Innenteile und der Medienkanäle beinhalten, und bei dem eine Vielzahl von Düsen (44) angeordnet ist, über die die Medien zur Behandlung der Außenflächen der Instrumente zuführbar sind.

17. Gerät nach Anspruch 16, bei dem die Düsen (44) an Teilen (3) des Kammergehäuses (43) angeordnet sind und die Kassette (12) einer oder mehreren mit Öffnungen (19) für den Zu- und Ablauf der Behandlungsmedien versehen ist.

18. Gerät nach Anspruch 17, bei dem die Düsen (44) an sich entlang der Kammer (2) erstreckenden Düsenarmen (3) angeordnet sind, die an einer Rückwand (43) der Kammer befestigt und so angeordnet sind, daß die Oberfläche der Instrumente (13) vollständig mit den Behandlungsmedien beaufschlagbar sind, wobei die Rückwand (15) der Kassette (12) mit Öffnungen (17) versehen ist, durch die bei Einschieben der Kassette in die Kammer die Düsenarme (3) hindurchgreifen.

19. Gerät nach Anspruch 18, bei dem die Düsenarme (3) in einem Winkel von 120° zueinander um die Instrumente (13) angeordnet sind.

20. Gerät nach Anspruch 19, bei dem die Düsenöffnungen (44) entlang der Düsenarme (3), bezogen auf eine durch die Längsachse verlaufende Bezugsebene, gegeneinander um einen kleinen Winkel versetzt angeordnet sind.

21. Gerät nach Anspruch 20, bei dem der Versatz so ausgerichtet ist, daß die Düsenöffnungen (44) alternierend ober- und unterhalb der Bezugsebene verlaufen.

22. Gerät nach einem der Ansprüche 18 bis 20, bei dem die Düsenarme (3) um die Instrumente (13) herum so angeordnet sind, daß für einen Adapter (16) die Düsen (44) von drei Düsenarmen (3) wirksam sind.

23. Gerät nach Anspruch 22, bei dem ein Düsenarm (3) zur Beaufschlagung mehrerer, auf Adapter (16) gehalteter Instrumente (13) vorgesehen ist.

24. Gerät nach einem der Ansprüche 15 bis 23, bei dem die Adapter (16) leicht wechselbar an der Rückwand (15) der Kassette (12) halterbar sind.

25. Gerät nach Anspruch 24, bei dem mindestens ein Adapter (16) Leitungskanäle (34, 36) zur Zuführung der Behandlungsmedien in das Innere der Instrumente (13) aufweist.

26. Gerät nach Anspruch 25, bei dem der Adapter (16) ein Steuerventil (32) beinhaltet, welches die Leitungskanäle(33, 35) für die Zufuhr der Behandlungsmedien bei nicht mit einem Instrument (13) belegtem Adapter verschließt.

27. Gerät nach Anspruch 24, bei dem mehrere Adapter (16) für unterschiedliche Instrumente (13) vorgesehen sind, wobei die Adapter bezüglich ihrer Halterung an der Gehäusewand (15) gleiche Anschlußmaße aufweisen.

28. Gerät nach einem der Ansprüche 15 bis 27, bei dem die Kassette (12) aus zwei durch Querteilung gebildeten und miteinander verbindbaren Gehäusehälften (12a, 12b) besteht, wobei die bodenseitige Gehäusehälfte (12a) die Adapter (16) trägt und die deckseitige Gehäusehälfte (12b) Führungselemente (4) zur Führung der Kassette (12) in der Kammer (2) des Gerätes aufweist.

29. Gerät nach einem der Ansprüche 18 bis 28, bei dem die Öffnungen (17) in der Kassette (12) für den Durchtritt der Düsenarme (3) von einem Abdeckmittel (20) verschlossen werden, wobei dem Abdeckmittel ein Steuermittel (20a; 22) zur Freigabe der Öffnungen zugeordnet ist.

30. Gerät nach Anspruch 29, bei dem das Abdeckmittel eine an der Rückwand (15) der Kassette (12) schwenkbar gehalterte Platte (20) ist, die einen Überstand (20a) aufweist, der bei Einführen der Kassette in die Kammer (3) an einer Gehäusekante (22) zu Anlage kommt und dabei die Platte im Sinne einer Freigabe der Öffnungen verstellt.

31. Gerät nach einem der vorangegangenen Ansprüche, bei dem die Kassette (12) aus einem sterilisierbaren Kunststoff besteht.

## Claims

1. Method for the hygienic preparation of medical, in particular dental, instruments with or without media channels and with or without movable inner portions, wherein in a single device (1) and in a chamber which can be sealed in a pressure-tight manner, the following method steps occur automatically in a preselectable process:
1.1 cleaning of the outer surfaces of the instruments (13, 14) and also, if applicable, of the media channels (27, 28) by means of a high-energy water jet that is directed at the instruments in a controlled manner, firstly with cold water, subsequently with preheated water, in which case the water jet is supplied in a pulsating manner and a gas under comparatively high pressure is admixed with the water in the interpulse periods;
1.2 intensive subsequent cleaning and disinfection of the outer surfaces and, if applicable, of the media channels (27, 28) and also of the movable inner portions (25) and their bearings (26) by means of a process of blowing off and out with steam at 60 to 100°C;
1.3 maintenance of the movable inner portions (25) and their bearings by injecting a dosed quantity of a lubricant;
1.4 sterilization of the instruments (13, 14) outside and inside by means of saturated steam;
1.5 drying and cooling of the instruments (13, 14) by means of a coolant.

2. Method according to claim 1, wherein intermediate drying by means of a coolant follows method step 1.2.

3. Method according to claim 1, wherein the maintenance is effected by means of an oil/air mixture.

4. Method according to claim 3, wherein the quantity of oil is automatically selected as a function of the adapted instrument (13).

5. Method according to claim 3, wherein the quantity of oil lies between 0.1 and 2 grammes per instrument and injection cycle.

6. Method according to claim 1, wherein as a result of intervening in the course of the method the latter can be interrupted after method step 1.3.

7. Method according to claim 1, wherein as a result of intervening in the course of the method it is possible to skip method step 1.4.

8. Method according to claim 1, wherein the drying and cooling is effected by means of compressed air.

9. Method according to claim 1, wherein the treatment room is evacuated before sterilization.

10. Method according to claim 1, wherein the drying of the instruments is vacuum-assisted.

11. Method according to one of the preceding claims, wherein the duration of the whole cycle preferably lies in the range between 10 and 20 minutes, with the individual cycles lasting on average:
3 minutes for method step 1.1
2 minutes for method step 1.2
0.5 minutes for method step 1.3
6.5 minutes for method step 1.4
3 minutes for method step 1.5.

12. Method according to claim 1, wherein the pulse frequency for the pulsating supply of the gas lies between 1 Hz and 10 Hz and preferably amounts to 3 Hz.

13. Method according to one of the preceding claims, wherein at least method steps 1.1 and 1.2 and, if applicable, method step 1.3 occur separately in terms of time one after the other for each instrument, preferably for instruments (13) which have media channels (27, 28) and moving inner portions (25, 26).

14. Device for carrying out the method according to one of claims 1 to 13, wherein provided in the chamber (2), which can be sealed in a pressure-tight manner, there is a fixedly arranged nozzle arrangement that has a plurality of nozzles (44), which are arranged around the instruments (13, 14) and are directed at the outer surfaces of the instruments, and which device has a microprocessor (50) which controls the water and gas supply in such a way that the water supply is effected in a pulsating manner and a gas under comparatively high pressure is admixed with the water in the interpulse periods.

15. Device according to claim 14, containing a cassette (12) which receives the instruments (13, 14) and which can be inserted into the sealable chamber (2) in which the method process runs.

16. Device according to claim 15, wherein provided in the cassette (12), at least for instruments (13) which have media channels (27, 28) and, if applicable, movable inner portions (25), there are adapters (16) which contain means (24) for holding the instruments and also means (33 to 36) for the supply of fluid and gaseous media for the treatment of the movable inner portions and the media channels and wherein there is arranged a plurality of nozzles (44) by way of which the media can be supplied for the treatment of the outer surfaces of the instruments.

17. Device according to claim 16, wherein the nozzles (44) are arranged on portions (3) of the chamber housing (43) and the cassette (12) is provided with one or more openings (19) for the supply and discharge of the treatment media.

18. Device according to claim 17, wherein the nozzles (44) are arranged on nozzle arms (3) which extend along the chamber (2) and which are secured and arranged on a rear wall (43) of the chamber in such a way that treatment media can act fully on the surface of the instruments (13), in which case the rear wall (15) of the cassette (12) is provided with openings (17) through which the nozzle arms (3) engage when the cassette is inserted into the chamber.

19. Device according to claim 18, wherein the nozzle arms (3) are arranged around the instruments (13) at an angle of 120° in relation to each other.

20. Device according to claim 19, wherein the nozzle openings (44) are arranged so as to be staggered in relation to each other by a small angle along the nozzle arms (3), relative to a reference plane extending through the longitudinal axis.

21. Device according to claim 20, wherein the staggered arrangement is aligned in such a way that the nozzle openings (44) extend alternately above and below the reference plane.

22. Device according to one of claims 18 to 20, wherein the nozzle arms (3) are arranged around the instruments (13) so that the nozzles (44) of three nozzle arms (3) are effective for one adapter (16).

23. Device according to claim 22, wherein one nozzle arm (3) is provided to act upon a plurality of instruments (13) held on adapters (16).

24. Device according to one of claims 15 to 23, wherein the adapters (16) can be held on the rear wall (15) of the cassette (12) in such a way that they can easily be exchanged.

25. Device according to claim 24, wherein at least one adapter (16) has line channels (34, 36) for the purpose of supplying the treatment media into the interior of the instruments (13).

26. Device according to claim 25, wherein the adapter (16) contains a control valve (32), which seals the line channels (33, 35) for the supply of the treatment media when the adapter is not occupied by an instrument (13).

27. Device according to claim 24, wherein a plurality of adapters (16) are provided for different instruments (13), in which case the adapters have the same connecting dimensions with regard to their holding support on the housing wall (15).

28. Device according to one of claims 15 to 27, wherein the cassette (12) consists of two housing halves (12a, 12b) which are formed by transverse division and can be connected together, in which case the housing half (12a) on the base side bears the adapters (16) and the housing half (12b) on the top side has guide elements (4) for guiding the cassette (12) in the chamber (2) of the device.

29. Device according to one of claims 18 to 28, wherein the openings (17) in the cassette (12) for the through-passage of the nozzle arms (3) are sealed by a covering means (20), in which case a control means (20a; 22) for the purpose of freeing the openings is associated with the covering means.

30. Device according to claim 29, wherein the covering means is a plate (20) which can be held such that it can be pivoted on the rear wall (15) of the cassette (12) and has a projection (20a) which when the cassette is inserted into the chamber (3) comes to rest against a housing edge (22) and thereby displaces the plate for the purpose of freeing the openings.

31. Device according to one of the preceding claims, wherein the cassette (12) consists of a sterilizable plastics material.

## Revendications

1. Dispositif de préparation hygiénique d'instruments médicaux, de dentisterie notamment, qui comportent ou non des canaux à fluides et qui comportent ou non des pièces intérieures mobiles, dans lequel les étapes de processus suivantes ont lieu automatiquement, dans un appareil unique (1) et dans une chambre qui peut être fermée hermétiquement, selon un processus que l'on peut choisir à l'avance :
1.1 nettoyage des surfaces extérieures des instruments (13, 14) ainsi que, le cas échéant, des canaux à fluides (27, 28) au moyen d'un jet d'eau à grande énergie dirigé de manière précise vers les instruments, tout d'abord avec de l'eau froide, puis avec de l'eau préchauffée, le jet d'eau étant envoyé par à-coups et un gaz sous haute pression étant additionné à l'eau au cours des intervalles entre impulsions,
1.2 nettoyage supplémentaire et désinfection intensifs des surfaces extérieures, et le cas échéant des canaux à fluides (27, 28) ainsi que des pièces intérieures mobiles (25) et de leurs supports (26), en détachant et en chassant les impuretés au moyen de vapeur d'eau à une température comprise entre 60 et 100 °C.
1.3 Entretien des pièces intérieures mobiles (25) et de leurs supports par injection d'une quantité dosée de lubrifiant,
1.4 stérilisation des instruments (13, 14), à l'extérieur et à l'intérieur, au moyen de vapeur d'eau saturée,
1.5 Séchage et refroidissement des instruments (13, 14) au moyen d'un fluide de refroidissement.

2. Procédé selon la revendication 1, dans lequel un séchage intermédiaire effectué au moyen d'un fluide de refroidissement a lieu à la suite de l'étape de processus 1.2.

3. Procédé selon la revendication 1, dans lequel l'entretien a lieu au moyen d'un mélange huile-air.

4. Procédé selon la revendication 3, dans lequel la quantité d'huile est choisie automatiquement en fonction de l'instrument (13) placé sur l'adaptateur.

5. Procédé selon la revendication 3, dans lequel la quantité d'huile est comprise entre 0,1 et 2 grammes par instrument et par cycle d'injection.

6. Procédé selon la revendication 1, dans lequel il est possible d'interrompre le déroulement du processus après l'étape de processus 1.3 en intervenant au cours dudit déroulement de processus.

7. Procédé selon la revendication 1, dans lequel il est possible de sauter l'étape de processus 1.4 en intervenant au cours du déroulement du processus.

8. Procédé selon la revendication 1, dans lequel le séchage et le refroidissement ont lieu au moyen d'air comprimé.

9. Procédé selon la revendication 1, dans lequel on fait le vide dans la chambre de traitement avant la stérilisation.

10. Procédé selon la revendication 1, dans lequel le vide contribue au séchage des instruments.

11. Procédé selon l'une des revendications précédentes, dans lequel le cycle complet dure de préférence de 10 à 20 minutes avec des cycles élémentaires qui durent en moyenne :
3 minutes pour l'étape de processus 1.1,
2 minutes pour l'étape de processus 1.2,
0,5 minute pour l'étape de processus 1.3,
6,5 minutes pour l'étape de processus 1.4,
et 3 minutes pour l'étape de processus 1.5.

12. Procédé selon la revendication 1, dans lequel la fréquence des impulsions d'envoi de gaz par à-coups se situe entre 1 Hz et 10 Hz et est de préférence de 3 Hz.

13. Procédé selon l'une des revendications précédentes, dans lequel, pour chaque instrument, et de préférence pour les instruments (13) qui possèdent des canaux à fluide (27, 28) et des pièces intérieures mobiles (25, 26), les étapes de processus 1.1 et 1.2 au moins, et le cas échéant l'étape de processus 1.3, se déroulent chronologiquement les unes après les autres en étant séparées.

14. Appareil de mise en oeuvre du procédé selon l'une des revendications 1 à 13, dans lequel, d'une part, il est prévu, dans la chambre (2) qui peut être fermée hermétiquement, un dispositif à buses fixe comportant une pluralité de buses (44) disposées autour des instruments (13, 14) et dirigées vers les surfaces extérieures des instruments, et qui, d'autre part, possède un microprocesseur (50) qui commande l'envoi d'eau et de gaz de sorte que l'envoi d'eau a lieu de manière discontinue et qu'un gaz sous haute pression soit additionné à l'eau dans les intervalles entre impulsions.

15. Appareil selon la revendication 14, comprenant une cassette (12) où sont placés les instruments (13, 14), laquelle peut être insérée dans la chambre pouvant être fermée (2) dans laquelle le processus se déroule.

16. Appareil selon la revendication 15, dans lequel il y a dans la cassette (12), au moins pour les instruments (13) qui possèdent des canaux à fluide (27, 28) et le cas échéant des pièces intérieures (25) mobiles, des adaptateurs (16) qui comportent des organes (24) de fixation des instruments ainsi que des organes (33 à 36) d'amenée de fluides liquides ou gazeux servant au traitement des pièces intérieures mobiles et des canaux à fluide, et dans lequel une pluralité de buses (44), par lesquelles peuvent passer les fluides de traitement des surfaces extérieures, sont disposées.

17. Appareil selon la revendication 16, dans lequel les buses (44) sont disposées sur des pièces (3) du boîtier de chambre (43), et dans lequel la cassette (12) est munie d'un ou de plusieurs orifices (19) d'amenée et d'écoulement des fluides de traitement.

18. Appareil selon la revendication 17, dans lequel les buses (44) sont ménagées sur des bras à buses (3) qui s'entendent le long de la chambre (2) et qui sont fixés à une paroi arrière (43) de la chambre en étant disposés de sorte que la totalité de la surface des instruments (13) puisse être atteinte par les fluides de traitement, la paroi arrière (15) de la cassette (12) étant pourvue d'orifices (17) au travers desquels les bras à buses (3) rentrent lors de l'introduction de la cassette dans la chambre.

19. Appareil selon la revendication 18, dans lequel les bras à buses (3) sont disposés autour des instruments (13) en ayant un écartement angulaire de 120° les uns par rapport aux autres.

20. Appareil selon la revendication 19, dans lequel les ouvertures de buse (44) sont décalées les unes par rapport aux autres d'un petit angle le long des bras à buses (3), et ce, par rapport à un plan de référence qui passe par l'axe longitudinal.

21. Appareil selon la revendication 20, dans lequel le décalage est orienté de sorte que les ouvertures de buse (44) se situent en alternance au-dessus et au-dessous du plan de référence.

22. Appareil selon l'une des revendications 18 à 20, dans lequel les bras à buses (3) sont disposés autour des instruments (13) de sorte que, pour chaque adaptateur (16), les buses (44) de trois bras à buses (3) ont une action effective.

23. Appareil selon la revendication 22, dans lequel il est prévu qu'un bras à buses (3) puisse atteindre plusieurs instruments (13) fixés sur des adaptateurs (16).

24. Appareil selon l'une des revendications 15 à 23, dans lequel les adaptateurs (16) peuvent être fixés à la paroi arrière (15) de la cassette (12) en étant facilement interchangeables.

25. Appareil selon la revendication 24, dans lequel au moins un adaptateur (16) possède des conduits (34, 36) d'amenée des fluides de traitement à l'intérieur des instruments (13).

26. Appareil selon la revendication 25, dans lequel l'adaptateur (16) comporte une soupape de commande (32) qui obture les conduits (33, 35) d'amenée de fluides de traitement lorsque l'adaptateur n'est occupé par aucun instrument (13).

27. Appareil selon la revendication 24, dans lequel plusieurs adaptateurs (16) sont prévus pour différents instruments (13) et possèdent des cotes de raccordement identiques en ce qui concerne leur fixation sur la paroi de boîtier (15).

28. Appareil selon l'une des revendications 15 à 27, dans lequel la cassette (12) est constituée de deux moitiés de boîtier (12a, 12b) qui sont formées par partage transversal et qui peuvent être assemblées, la moitié inférieure de boîtier (12a) portant les adaptateurs (16) et la moitié supérieure de boîtier (12b) comportant des éléments de guidage (4) servant à guider la cassette (12) à l'intérieur de la chambre (2) de l'appareil.

29. Appareil selon l'une des revendications 18 à 28, dans lequel les orifices (17) de la cassette (12) qui sont destinés au passage des bras à buses (3) sont obturés par un organe de recouvrement (20), un organe de commande (20a ; 22) destiné à dégager l'accès aux orifices étant associé à l'organe de recouvrement.

30. Appareil selon la revendication 29, dans lequel l'organe de recouvrement est une plaque (20) qui est fixée à la paroi arrière (15) de la cassette (12) de manière à pouvoir basculer et qui comporte une saillie (20a) qui, lors de l'introduction de la cassette dans la chambre (2), prend appui sur un bord de boîtier (22) et, ce faisant, déplace la plaque en dégageant l'accès aux orifices.

31. Appareil selon l'une des revendications précédentes, dans lequel la cassette (12) est faite à partir d'une matière plastique qui peut être stérilisée.
